(19) Europäisches Patentamt
European Patent Office
Office européen des brevets

(11) Numéro de publication: **0 374 002 A1**

(12) # DEMANDE DE BREVET EUROPEEN

(21) Numéro de dépôt: **89403374.5**

(22) Date de dépôt: **06.12.89**

(51) Int. Cl.5: **C07D 413/12, A01N 47/24, C07D 263/26**

(30) Priorité: **14.12.88 FR 8816435**

(43) Date de publication de la demande: **20.06.90 Bulletin 90/25**

(84) Etats contractants désignés: **CH DE GB IT LI**

(71) Demandeur: **SOCIETE NATIONALE DES POUDRES ET EXPLOSIFS**
**12, quai Henri IV**
**F-75181 Paris Cédex 04(FR)**

Demandeur: **SOCIETA ITALIANA PRODOTTI CHIMICI E PER L'AGRICOLTURA (SIPCAM)**
**Viale Gian Galeazzo, 3**
**I-20136 Milano(IT)**

(72) Inventeur: **Denarie, Michel**
**30, Parc d'Ardenay**
**F-91120 Palaiseau(FR)**
Inventeur: **Formigoni, Attilio**
**Via Pietro Micca, 11**
**I-20025 Legnano Milano(IT)**
Inventeur: **Senet, Jean-Pierre**
**79, rue de la Gare Herbeauvilliers Buthiers**
**F-77760 La Chapelle La Reine(FR)**

(74) Mandataire: **Pech, Bernard et al**
**Sté nationale des poudres et explosifs 12, quai Henri IV**
**F-75181 Paris Cédex 04(FR)**

(54) **Dérivés du méthylcarbamate de dihydro-2,3 diméthyl-2,2 benzofurannyle-7, leur procédé de préparation, les compositions pesticides les contenant et dérivés chlorosulfénylés utiles pour les préparer.**

(57) L'invention concerne des dérivés de formule (I) :

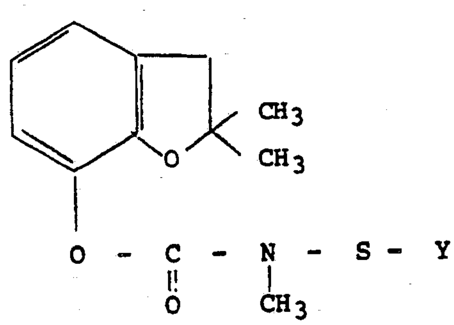

(I)

dans laquelle Y représente le radical one-2 oxazolidinyl-3, substitué ou non.

Elle concerne également les composés de formule (III) Y-S-Cl dans laquelle Y a la signification précédente et leur procédé de préparation par réaction des composés de formule Y-H ou de leurs dérivés N-trialkylsilylés avec le dichlorure de soufre.

Les composés de formule (I) sont obtenus par réaction des composés de formule (III) avec le Carbofuran.

Ils sont utilisés comme matière active de compositions pesticides.

EP 0 374 002 A1

**Dérivés du méthylcarbamate de dihydro-2,3 diméthyl-2,2 benzofurannyle-7, leur procédé de préparation, les compositions pesticides les contenant et dérivés chlorosulfénylés utiles pour les préparer.**

La présente invention concerne de nouveaux dérivés du méthylcarbamate de dihydro-2,3 diméthyl-2,2 benzofurannyle-7, leur procédé de préparation, les compositions pesticides les contenant comme matière active et un procédé de lutte contre les parasites par application de ces nouveaux composés. Elle concerne également les dérivés chlorosulfénylés utiles pour les fabriquer et leurs procédés de préparation.

Il est connu que le dihydro-2,3 diméthyl-2,2 benzofurannyle-7 également dénommé "carbofuran" et certains de ses dérivés tels que les dérivés N-aminosulfénylés possèdent une activité pesticide.

Cependant ou bien ces composés possèdent une toxicité élevée vis à vis des animaux à sang chaud ou bien lorsqu'ils sont moins toxiques leur activité pesticide est plus faible et en particulier leur activité systémique.

Par conséquent il était important de disposer de nouveaux composés ayant une excellente activité pesticide, un pouvoir systémique accru sans être plus toxiques.

L'objet de la présente invention concerne des nouveaux composés qui possèdent les caractéristiques recherchées et ne présentent pas les inconvénients des produits antérieurs.

Les nouveaux composés selon l'invention répondent à la formule suivante :

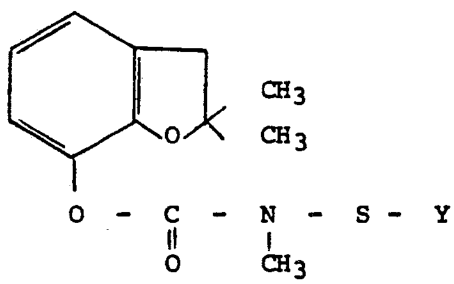

(I)

dans laquelle Y représente le radical one-2 oxazolidinyl-3, substitué ou non.

Les composés de formule (I) sont de nouveaux composés qui n'ont pas été décrits dans la littérature.

La combinaison du cycle one-2 oxazolidinyle et des autres groupes fonctionnels donne à ces composés leurs propriétés particulières.

Le cycle one-2 oxazolidinyl-3 peut être non substitué ou porter de un à quatre substituants identiques ou différents très variés choisis en particulier parmi :

- les radicaux alkyles en $C_1$ à $C_8$, linéaires ou ramifiés, substitués ou non par des atomes d'halogène, des groupes alkylthio en $C_1$ à $C_4$, des groupes hydroxyles,
- les radicaux alcoxy en $C_1$ à $C_4$, linéaires ou ramifiés,
- les radicaux alcoxycarbonyle en $C_1$ à $C_4$, linéaires ou ramifiés, - le groupe carboxyle,
- les radicaux cycloalkyles en $C_3$ à $C_6$,
- le radical phényle porteur ou non d'un ou plusieurs substituants choisis parmi les atomes d'halogène, les groupes nitro ou cyano, les radicaux alkyles ou alcoxy en $C_1$ à $C_4$, le radical trifluorométhyle,

Le cycle one-2 oxazolidinyl-3 peut également être condensé avec un cycle à 5 ou 6 chaînons, saturé ou non, hétérocyclique ou non, l'hétéroatome étant choisi parmi O, S et N, le système cyclique pouvant être substitué ou non par des atomes d'halogène, des radicaux alkyles ou halogénoalkyles en $C_1$ à $C_4$, linéaires ou ramifiés.

Les composés préférés sont les composés de formule (I) dans laquelle Y représente :

- le cycle one-2 oxazolidinyl-3 non substitué ou substitué par un à quatre groupes identiques ou différents choisis parmi :

. les radicaux alkyles en $C_1$ à $C_8$, linéaires ou ramifiés, substitués ou non par des atomes de fluor, chlore ou brome, le radical méthylthio, le groupe hydroxyle,

. les radicaux méthoxy et éthoxy,

. les radicaux alkoxycarbonyle en $C_1$ à $C_4$,

. le groupe carboxyle,

. les radicaux cycloalkyle en $C_3$ ou $C_6$,

. le radical phényle porteur ou non d'un ou de plusieurs substituants choisis parmi les atomes de chlore, brome ou fluor, le groupe nitro ou cyano,les radicaux méthyle et méthoxy,
- le cycle one-2 oxazolidinyl-3 condensé avec un cycle à 6 chaînons saturé ou non, hétérocyclique ou non, l'hétéroatome étant choisi parmi O, S et N, le système cyclique étant substitué ou non par des groupes choisis parmi les atomes de fluor, chlore ou brome, les radicaux alkyles en $C_1$ à $C_3$, linéaires ou ramifiés, les radicaux mono-, di- ou tri-, chloro-, bromo- ou fluorométhyle.

Les composés particulièrement appréciés sont les composés de formule (I) dans laquelle Y représente le cycle one-2 oxazolidinyl-3, non substitué ou substitué par un à quatre groupes identiques ou différents choisis parmi les radicaux alkyles en $C_1$ à $C_4$, linéaires ou ramifiés.

L'invention concerne également les composés nouveaux de formule
Y - S - Cl (III)
dans laquelle Y a la signification précédente.

Ces composés n'ont jamais été décrits précédemment. Ils sont très utiles en tant que produits intermédiaires et en particulier pour la synthèse des composés de formule (I).

Ils peuvent être préparés par des procédés variés et en particulier par réaction des 2-oxazolidones de formule Y-H (IV) dans laquelle Y a la signification précédente, avec le dichlorure de soufre $SCl_2$ en présence d'un composé basique, par exemple une amine tertiaire telle que la pyridine, la triéthylamine, la lutidine, la picoline, la N,N-diméthylaniline.

La réaction a généralement lieu dans un milieu solvant inerte vis à vis des reactifs. Comme exemple de solvants utiles ou peut citer les solvants aliphatiques halogénés, les éthers tels que le tétrahydrofuranne, le dioxanne, l'éther diéthylique et les solvants aromatiques. De préférence on utilise le chlorure de méthylène ou l'éther diéthylique.

La température de la réaction est généralement comprise entre - 10° et 50°C et de préférence entre 0° et 20°C.

Les composés sont généralement mis à réagir en quantité voisine de la stoechiométrie. On peut également utiliser un léger excès de $SCl_2$.

Les 2-oxazolidones (IV) sont des composés connus et bien décrits dans la littérature ainsi que leur procédé de préparation (cf. M. E. Dyen et D. Swern, Chem. Review, 67, p. 197-246, 1967 ou V.A. Pankratov et al, Russian Chemical Reviews - 52 (6) P.576-579, 1983).

On peut également préparer les composés nouveaux de formule (III) par réaction des dérivés N-trialkylsilylés des 2-oxazolidones de formule (IV), le radical alkyl ayant de 1 à 4 atomes de carbone, avec le dichlorure de soufre.

Les dérivés N-trialkylsilylés sont eux-mêmes des dérivés connus que l'on trouve dans le commerce ou que l'on prépare de manière analogue à des procédés connus par exemple comme décrit dans le brevet FR 2 218 342.

La réaction des N-trialkylsilyl-2-oxazolidones avec le dichlorure de soufre a généralement lieu dans un milieu solvant inerte vis à vis des réactifs,tel que celui décrit précédemment. De préférence on utilise le chlorure de méthylène ou l'éther diéthylique.

Les composés sont mis à réagir en quantité voisine de la stoechiométrie. Un léger excès de $SCl_2$ peut également être utilisé.

La température de la réaction est généralement compris entre - 10° et 50° C et de préférence entre 0° et 20° C.

Les composés de formule (III) précédement obtenus permettent de préparer de façon simple les composés de formule (I) par réaction avec le composé de formule (II) :

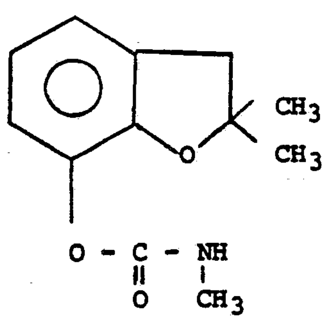

(II)

en présence d'un composé basique.

Le schéma réactionnel est le suivant :

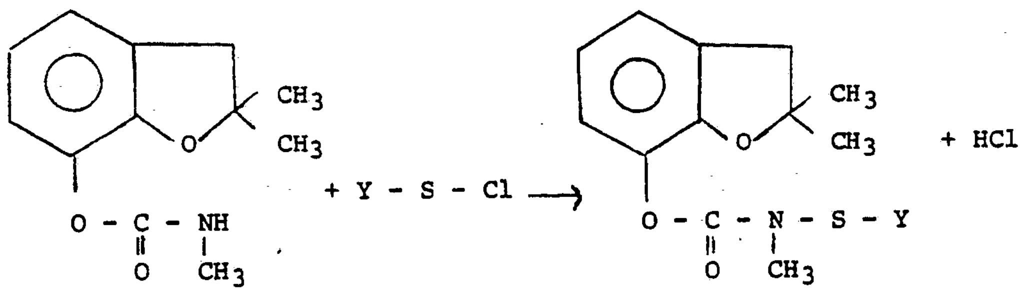

La réaction est généralement effectuée dans un milieu solvant inerte vis à vis des réactifs.

Comme exemple de solvants utiles on peut citer les solvants aliphatiques chlorés tels que le dichlorométhane, le chloroforme, le dichloro-1,2 éthane, les solvants aromatiques tels que le toluène, les xylènes, le chlorobenzène, les éthers cycliques tels que le tétrahydrofuranne et le dioxanne.

De préférence le milieu solvant est anhydre.

Le composé de formule (II) utilisé comme matière de départ est un composé connu, qui se trouve dans le commerce.

Les proportions des composés de formule (II) et (III) ne sont pas critiques et peuvent varier. Généralement on utilise une quantité du composé de formule (III) comprise entre 1 et 2 moles par mole de composé de formule (II) et de préférence entre 1 et 1,2 moles.

La réaction a lieu en présence d'un composé basique. Comme exemples de composés basiques utiles on peut citer les bases minérales telles que les hydroxydes et carbonates alcalins et comme bases organiques les amines tertiaires telles que la triéthylamine, la tributylamine, la N,N-diméthylaniline et les bases pyridiniques telles que la pyridine et la picoline.

On utilise généralement le composé basique en quantité suffisante pour fixer l'acide chlorhydrique qui se forme au cours de la réaction. Cette quantité est généralement comprise entre 1 et 10 moles par mole du composé de formule (II) et de préférence entre 1 et 1,6 moles.

La température de la réaction est généralement comprise entre - 10° C et 50° C, de préférence entre 0° C et 35° C.

La réaction dure généralement de 2 h à 48 h.

Le composé de formule (I) est généralement récupéré après filtration et élimination du solvant et purifié facilement par des procédés classiques tels que par extraction, recristallisation ou chromatographie sur colonne.

On a découvert que les nouveaux composés de formule (I) ont une excellente activité pesticide à large spectre et peuvent être utilisés dans la lutte contre des parasites, des insectes, des acariens, des myriapodes et des nématodes très divers, à un stade quelconque de leur développement tel que sous forme d'oeufs, de larves, de nymphes, de chrysalides et d'adultes, nuisibles aux végétaux et aux animaux et qu'ils possèdent surtout une remarquable activité systémique.

En particulier les composés selon l'invention possèdent une activité insecticide contre des insectes nuisibles appartenant aux ordres des lépidoptères, des diptères, des hémiptères, des coléoptères par exemple Spodoptera littoralis, Musca domestica, Aphis fabae, Tribolium confusum, Calandra granaria, et ils sont aussi actifs contre les acariens et les myriapodes dangereux pour les plantes et animaux et contre les nématodes parasites des plantes.

Ils sont particulièrement utiles dans la lutte contre les insectes, les acariens, les nématodes et les myriapodes des cultures de betteraves, maïs, pommes de terre, soja, colza, tournesol, sorgho, coton, canne à sucre, bananes, riz, blé, vigne, cultures fruitières, maraîchères ou ornementales, aussi bien pour les traitements des parties aériennes que pour les traitements du sol.

Les composés pesticides de formule (I) peuvent être utilisés tels quels ou avec des supports inactifs et/ou avec des surfactants ou solvants appropriés inertes vis à vis des matières actives, qui servent habituellement à la préparation des compositions pesticides.

Les compositions pesticides se préparent de façon connue en soi par exemple par dissolution, dispersion, mise en suspension, mélange, imprégnation, adsorption ou fixation et peuvent se présenter sous des formes très variées telles que des poudres, granulés, comprimés pâtes, émulsions, solutions, suspensions, huiles, compositions de revêtement, mousses à pulvériser, aérosols, micro-capsules obtenues, selon les techniques connues de l'homme de l'art.

La teneur en composé de formule (I) matière active de ces compositions est généralement comprise

entre 0,1 et 99 % en poids, de préférence entre 0,5 et 90 %.

D'autres ingrédients actifs peuvent être ajoutés dans les compositions par exemple d'autres insecticides, des nématocides, des régulateurs de croissance, des herbicides, des fongicides, des fertilisants, afin d'élargir leur champ d'application et les adapter à des circonstances particulières.

Les formulations suivantes peuvent par exemple être utilisées :

1. Pour applications foliaires ou aux animaux : des solutions liquides émulsionnables dans l'eau, des suspensions de solides dans l'eau ou dans des liquides organiques (pâtes fluides ou "flowables"), des microémulsions prêtes à l'emploi, des poudres mouillables dans l'eau ou des poudres pour pulvérisation telles quelles à sec.

2. Pour application dans le sol : des formulations granulaires applicables sur toute la surface du sol ou localisées dans les sillons des semis, des formulations en poudres ou les mêmes formulations ci-dessus indiquées pour les applications foliaires, avec incorporation dans le sol après application sur la surface du sol.

Les exemples qui suivent illustrent l'invention sans toutefois la limiter :

**Exemple 1** : Préparation du N-(one-2 oxazolidinyl-3-sulfényl)-N-méthylcarbamoyloxy 2,3-dihydro-2,2-diméthyl-7-benzofuranne (2)

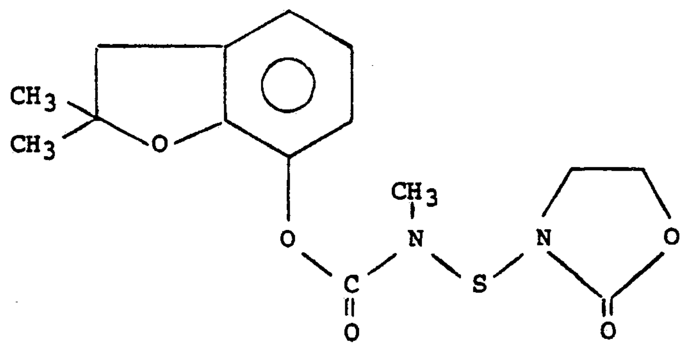

(2)

A : Préparation de la 3-chlorosulfényl-2-oxazolidone (1)

A une solution de dichlorure de soufre $SCl_2$ (13,4 g; 0,13 mol) dans l'éther diéthylique anhydre (50 ml) on ajoute lentement, à 0° C, une solution de 3-triméthylsilyl-2-oxazolidone (20 g ; 0,125 mol) dans l'éther diéthylique (30 ml). On laisse revenir la température du mélange réactionnel à 20° C et on maintient 30 minutes sous agitation. Après filtration, le solvant est éliminé sous pression réduite (15 mm Hg) et chauffage au bain-marie (30° C). On recueille ainsi le composé voulu sous forme d'un liquide fluide jaune-orangé (17,5 g, Rendement 91 %).

B : Réaction de la 3-chlorosulfényl-2-oxazolidone avec le N-méthylcarbamate de dihydro-2,3 diméthyl-2,2 benzofurannyle-7.

A une solution de N-méthylcarbamate de dihydro-2,3 diméthyl-2,2 benzofurannyle-7 (13 g ; 0,059 mol) dans le chlorure de méthylène (70 ml) on ajoute le composé (1) (10 g, 0,065 mol) en solution dans le chlorure de méthylène (15 ml) ; puis goutte à goutte, en maintenant la température inférieure à 10° C, de la pyridine (7 g, 0,088 mol). Le mélange réactionnel est agité 24 heures à température ambiante. On filtre le précipité et le solvant est éliminé par distillation sous pression réduite. L'huile obtenue est reprise par du benzène (50 mol), la suspension résultante filtrée et le benzène éliminé par distillation. On obtient ainsi une huile de couleur brune visqueuse qui contient essentiellement le composé (2). Une fraction de cette huile est purifiée par chromatographie sur une colonne de gel de silice en utilisant comme éluant un mélange d'hexane et d'acétate d'éthyle (7/3 ; v/v) pour donner le composé (2) sous forme d'un solide blanc.

Point de fusion : 114° C

RMN $^1H$ dans $CDCl_3$

δ 1,45 ppm (s, 6H)
δ 3,05 ppm (s, 2H)
δ 3,55 ppm (s, 3H)
δ 4,1 ppm (t, 2H)
δ 4,50 ppm (t, 2H)
δ 6,7-7,05 ppm (m, 3H)

| Analyse élémentaire pour $C_{15}H_{18}N_2O_5S$ (338,38) | | | | | |
|---|---|---|---|---|---|
| | C | H | N | O | S |
| Trouvé (%) | 53,24 | 5,36 | 8,28 | 23,64 | 9,47 |
| Théorique (%) | 53,16 | 5,36 | 8,11 | 23,75 | 9,58 |

**Exemple 2** : Préparation du N-(4,4-diméthyl one-2 oxazolidinyl-3 sulfényl)-N-méthylcarbamoyloxy-2,3-dihydro-2,2-diméthyl-7-benzofuranne (4)

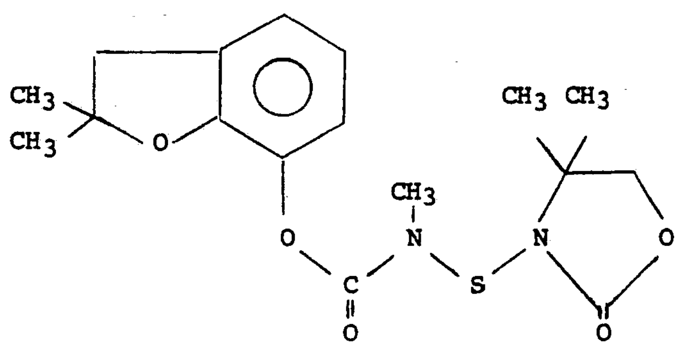

(4)

A : Préparation de la 3-triméthylsilyl-4,4-diméthyl-2-oxazolidone

A une solution de 4,4-diméthyl-2-oxazolidone (17,3 g ; 0,15 mole) dans le tétrahydrofuranne anhydre (150 ml) on ajoute de la triéthylamine (16,7 g ; 0,165 mol) en solution dans le tétrahydrofuranne (20 ml). La solution est refroidie à 0° C et on coule le triméthylchlorosilane (17,9 g ; 0,165 mol) en maintenant la température entre 0 et 5° C. On laisse alors la température revenir à 20° C et on maintient l'agitation pendant 1 heure. Le mélange réactionnel est filtré sous azote. Le chlorhydrate de triéthylamine est lavé avec du tétrahydrofuranne et le solvant est évaporé sous pression réduite (15 mm Hg) et chauffage au bain-marie (40° C). On recueille ainsi un liquide légèrement coloré (jaune).
La distillation conduit à un liquide incolore de point d'ébullition Eb = 129° C (15 mm Hg). Rendement 74,6 %.

B : Préparation de la 3-chlorosulfényl-4,4-diméthyl-2-oxazolidone (3)

En procédant selon l'exemple 1A et en remplaçant la 3-triméthylsilyl-2-oxazolidone par le composé obtenu en A on obtient le composé souhaité sous forme d'un liquide jaune (Rendement 95 %).

C : Réaction de la 3-chlorosulfényl-4,4-diméthyl-2-oxazolidone avec le carbofuran

On utilise le mode opératoire décrit dans l'exemple 1B en remplacant le composé (1) par le composé (3). On obtient le produit brut sous forme d'une huile orange visqueuse.

Une fraction de cette huile est purifiée par chromatographie sur colonne de gel de silice en utilisant comme éluant un mélange d'hexane et d'éther diéthylique (7/3 ; v/v) pour obtenir le composé de formule (4) sour forme d'un solide blanc.
Point de fusion : 99-101 °C
RMN $^1H$ dans $CDCl_3$
δ 1,45 ppm (s, 6H)
δ 1,47 ppm (s, 6H)
δ 3,05 ppm (s, 2H)
δ 3,55 ppm (s, 3H)
δ 4,1 ppm (s, 2H)
δ 6,7-7,05 ppm (m, 3H)

| Analyse élémentaire pour $C_{17}H_{22}N_2O_5S$ (366,44) | | | |
|---|---|---|---|
| | C | H | N |
| Trouvé (%) | 55,72 | 6,05 | 7,64 |
| Théorique (%) | 55,68 | 5,99 | 7,31 |

**Exemple 3** : Préparation du N-(5-méthyl one-2 oxazolidinyl-3-sulfényl)-N-méthylcarbamoyloxy-2,3-dihydro-2,2-diméthyl-7-benzofuranne (6)

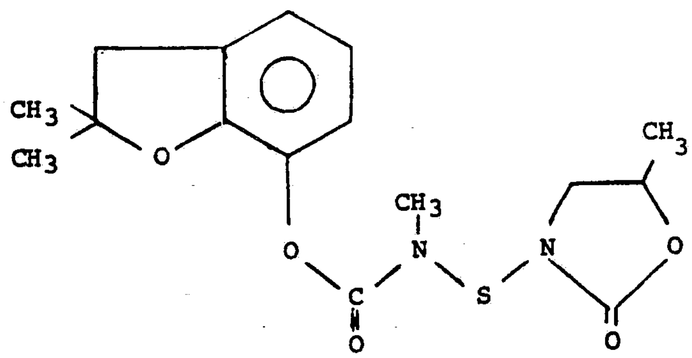

(6)

A : Préparation de la 3-chlorosulfényl-5-méthyl-2-oxazolidone (5)

En suivant le mode opératoire de l'exemple 1A et en remplaçant la 3-triméthylsilyl-2-oxazolidone par la 3-triméthylsilyl-5-méthyl-2-oxazolidone, on obtient le 3-chlorosulfényl-5-méthyl-2-oxazolidone sous forme d'un liquide fluide jaune-orangé (Rendement 92,9 %).

B : Réaction de la 3-chlorosulfényl-5-méthyl-2-oxazolidone avec le carbofuran

On prépare le composé (6) de la même façon que dans l'exemple 1B en remplacant le composé (1) par le composé (5). On obtient le produit brut sous forme d'une huile brune visqueuse.

Après purification par chromatographie sur colonne de gel de silice en utilisant comme éluant un mélange d'hexane et d'éther diéthylique (7/3 ; v/v) on obtient une huile incolore très visqueuse.
RMN $^1H$ dans $CDCl_3$
δ 1,40 ppm (d, 3H)
δ 1,45 ppm (s, 6H)
δ 3,0 ppm (s, 2H)

δ 3,50 ppm (s, 3H)
δ 3,6-4,9 ppm (m, 3H)
δ 6,7-7,1 ppm (m, 3H)

| Analyse élémentaire pour $C_{16}H_{20}N_2O_5S$ (352,41) | | | | |
|---|---|---|---|---|
| | C | H | N | S |
| Trouvé (%) | 54,53 | 5,72 | 7,95 | 9,10 |
| Théorique (%) | 54,48 | 5,88 | 8,08 | 9,22 |

Exemple 4

Les composés (2) et (6) obtenus dans les exemples précédents sont formulés sous forme de granules, avec micronisation préalable de la matière active (2) solide dans un moulin approprié jusqu'à une finesse des particules comprise entre 2 et 20 microns, et par mélange successif des substances actives, des inertes granulaires et des adjuvants selon les formulations suivantes :

| | (I) | (II) |
|---|---|---|
| Composé (2) solide | 5 kg | - |
| Composé (6) liquide | - | 5 kg |
| Résine 1413 * | 1 kg | - |
| Dipropylène glycol | - | 2 kg |
| Sable silicique granulaire de 0,4-0,8 mm | 94 kg | - |
| Gypse granulaire de 0,5-1 mm | - | 93 kg |
| Total | 100 kg | 100 kg |

* vendu par la Société TEGO - Lodi (Italie)

Dans un mélangeur rotatif on obtient des formulations granulaires prêtes à l'emploi.

Exemple 5

Le composé (6) obtenu précédemment est mélangé dans un mélangeur pour liquides avec les inertes et les adjuvants suivants :

| | (III) |
|---|---|
| Composé (6) liquide | 25 kg |
| SOITEM 849 * | 3 kg |
| SOITEM 251 * | 4 kg |
| Cyclohexanone | 10 kg |
| Xylène | 58 kg |
| Total | 100 kg |

* vendu par la Société SOITEM - Milan (Italie)

On obtient une solution liquide émulsionnable dans l'eau prête à l'emploi.

Exemple 6

Le composé (4) exemplifié précédemment, en poudre fine, est mélangé avec les inertes et les adjuvants suivants :

| | (IV) |
|---|---|
| Composé (4) solide | 35,0 kg |
| Propylène glycol | 7,0 kg |
| SOITEM 8FL * | 3,0 kg |
| ANTIFOAM AF ** | 0,1 kg |
| RHODOPOL 23 ** | 0,3 kg |
| Benzoate de sodium | 0,1 kg |
| Eau | 54,5 kg |
| Total | 100,0 kg |

* vendu par la Société SOITEM - Milano (Italie)

** vendu par la Société RHONE-POULENC SA - PARIS (France)

Le mélange est ensuite passé dans un microniseur colloïdal à perle pour obtenir une suspension fluide dans l'eau prête à l'emploi.

Exemple 7

On a testé l'activité insecticide par contact in vitro en déposant dans des boîtes de Petri de 10 cm de diamètre, 1 ml de solution acétonique des pesticides à tester, contenant des doses décroissantes de matières actives. Ensuite on dépose dans chaque boîte de Petri 25 insectes adultes de Calandra granaria. Trois essais sont effectués pour chaque dose de matière active. Après 2 heures et 48 heures on observe le pourcentage de mortalité des insectes.

Les résultats sont rassemblés dans le tableau I. A titre comparatif on a indiqué également dans ce tableau les résultats obtenus avec un composé connu.

TABLEAU I

| COMPOSES | Dose de matière active | Calandra granaria (Adultes) | |
|---|---|---|---|
| | ppm | % de mortalité après | |
| | | 2H | 48H |
| 2 | 1,6 | 93 | 100 |
| | 0,4 | 5 | 100 |
| | 0,1 | 0 | 73 |
| 4 | 1,6 | 0 | 100 |
| | 0,4 | 0 | 90 |
| | 0,1 | 0 | 22 |
| 6 | 1,6 | 75 | 100 |
| | 0,4 | 0 | 100 |
| | 0,1 | 0 | 30 |
| Benfuracarb | 1,6 | 0 | 100 |
| | 0,4 | 0 | 70 |
| | 0,1 | 0 | 0 |

Exemple 8

On a testé l'activité insecticide par contact sur plantes en pulvérisant 2 ml de dispersion aqueuse d'une solution acétonique contenant des doses décroissantes de matières actives dans une tour de Potter sur des feuilles de tabac infestées par 10 larves de 1 cm de Spodoptera littoralis disposées dans des boîtes de Petri de 10 cm de diamètre.

Trois essais sont effectués pour chaque dose de matières actives.

Les résultats exprimés en pourcentage de mortalité de Spodoptora littoralis après 48 heures pour les composés selon l'invention et à titre comparatif pour un composé connu sont indiqués dans le tableau II.

Tableau II

| COMPOSES | Spodoptera littoralis (larves) : % de mortalité après 48 heures | | |
|---|---|---|---|
| | Doses de matière active | | |
| | 1000 ppm | 250 ppm | 62,5 ppm |
| 2 | 100 | 80 | 37 |
| 4 | 100 | 80 | 40 |
| 6 | 100 | 87 | 43 |
| Benfuracarb | 100 | 80 | 30 |

Exemple 9

On a testé l'activité insecticide par contact sur plante par traitement foliaire.

On traite 3 plants de féverole infestés de nombreuses larves et d'adultes d'Aphis fabae avec 25 ml d'émulsion ou de suspension aqueuse des matières actives à doses décroissantes, dans une chambre de pulvérisation avec les plants en pots de 15 cm de diamètre sur plateau tournant.

Les résultats exprimés en pourcentage de mortalité pour les composés selon l'invention et pour un composé connu sont indiqués dans le tableau III.

TABLEAU III

| COMPOSES | Aphis fabae sur féverole (Adultes et larves) % de mortalité après 48 heures | | |
|---|---|---|---|
| | Doses de matières active | | |
| | 25 ppm | 6,25 ppm | 1,56 ppm |
| 2 | 100 | 92 | 11 |
| 4 | 100 | 65 | 0 |
| 6 | 100 | 59 | 0 |
| Benfuracarb | 100 | 91 | 9 |

Exemple 10

On a testé l'activité insecticide systémique par traitement d'un sol type limon-sableux avec incorporation à 5 cm de profondeur de doses décroissantes de matières actives, avant le semis, dans 3 pots de 15 cm de diamètre semés avec la féverole, qui vient d'être infestée par de nombreuses larves et adultes d'Aphis fabae, soit 13 jours, soit 23 jours après le semis.

Les résultats exprimés en pourcentage de mortalité d'Aphis fabae après 48 heures de nourriture sur les plantes, 13 et 23 jours après le semis, pour différentes doses de matière active (m.a.) calculées à l'hectare sont rassemblés dans le tableau IV.

TABLEAU IV

| COMPOSES | Doses m.a. kg/ha | Aphis fabae sur féverole % de mortalité à 48 heures par voie systémiqueè<br>Nombre de jours après traitement du sol<br>13 | 23 |
|---|---|---|---|
| 2 | 2<br>0,5<br>0,125 | 100<br>85<br>30 | 100<br>91<br>13 |
| 4 | 2<br>0,5<br>0,125 | 100<br>14<br>0 | 100<br>100<br>0 |
| 6 | 2<br>0,5<br>0,125 | 100<br>100<br>0 | 100<br>100<br>7 |
| Carbofuran | 2<br>0,5<br>0,125 | 100<br>86<br>0 | 100<br>82<br>0 |

Exemple 11

On a testé l'activité insecticide systémique par traitement d'un sol type limon-sableux avant repiquage en pots de 15 cm de diamètre, de 3 plants de tabac infestés, 9 jours et 29 jours après, par 10 larves de 1 cm de Spodoptera littoralis.

Les résultats exprimés en pourcentage de mortalité après 48 heures de nourriture sur les plantes 9 et 29 jours après le repiquage, pour différentes doses de matières actives sont rassemblées dans le tableau V.

TABLEAU V

| COMPOSES | Doses m.a. kg/ha | Spodoptera littoralis sur tabac : (larves) % de mortalité à 48 heures par voie systémique | |
|---|---|---|---|
| | | Nombre de jours après traitement du sol | |
| COMPOSES | kg/ha | 9 | 29 |
| 2 | 8<br>2<br>0,5 | 100<br>100<br>70 | 97<br>60<br>7 |
| 4 | 8<br>2<br>0,5 | 100<br>93<br>30 | 100<br>17<br>0 |
| 6 | 8<br>2<br>0,5 | 100<br>83<br>63 | 100<br>53<br>30 |
| Benfuracarb | 8<br>2<br>0,5 | 100<br>100<br>77 | 100<br>77<br>20 |

Exemple 12

On a testé l'activité insecticide systémique par traitement de semences de féverole enrobées avec des doses décroissantes de matières actives et semées dans 3 pots de 15 cm de diamètre en sol limoneux-sableux.

19 et 20 jours après le semis, on infeste les plantes avec de nombreuses larves et adultes de Aphis fabae et on observe chaque fois après 48 heures la mortalité des insectes.

Les résultats exprimés en pourcentage de mortalité pour différentes doses de matière active, 19 et 29 jours après le semis sont rassemblés dans le tableau VI.

TABLEAU VI

| COMPOSES | Doses kg m.a. / 100 kg semences | Aphis fabae sur féverole % de mortalité à 48 heures par voie systémique | |
|---|---|---|---|
| | | nombre de jours après traitement des semences | |
| | | 19 | 29 |
| 2 | 0,128<br>0,032<br>0,008 | 100<br>82<br>0 | 100<br>93<br>10 |
| 4 | 0,128<br>0,032<br>0,008 | 72<br>40<br>0 | 12<br>0<br>0 |
| 6 | 0,128<br>0,032<br>0,008 | 98<br>59<br>13 | 100<br>91<br>65 |
| Carbosulfan | 0,128<br>0,032<br>0,008 | 94<br>50<br>3 | 100<br>29<br>0 |

**Revendications**

1. Dérivés du méthylcarbamate de dihydro-2,3 diméthyl-2,2 benzofurannyle-7 caractérisés en ce qu'ils répondent à la formule

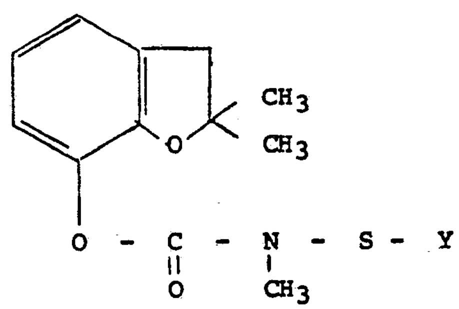

(I)

dans laquelle Y représente le radical one-2 oxazolidinyl-3, substitué ou non.

2. Composés selon la revendication 1 caractérisés en ce que Y représente :
- le radical one-2 oxazolidinyl-3,
- le radical one-2 oxazolidinyl-3 porteur de un à quatre substituants identiques ou différents, choisis parmi :
- les radicaux alkyles en $C_1$ à $C_8$, linéaires ou ramifiés, substitués ou non par des atomes d'halogène, des groupes alkylthio en $C_1$ à $C_4$, des groupes hydroxyles,
- les radicaux alcoxy en $C_1$ à $C_4$, linéaires ou ramifiés,
- les radicaux alcoxycarbonyle en $C_1$ a $C_4$, linéaires ou ramifiés,
- le groupe carboxyle,
- les radicaux cycloalkyles en $C_3$ à $C_6$,
- le radical phényle porteur ou non d'un ou plusieurs substituants choisis parmi les atomes d'halogène, les groupes nitro ou cyano, les radicaux alkyles et alcoxy en $C_1$ à $C_4$, le radical trifluorométhyle,
- le radical one-2 oxazolidinyl-3 condensé avec un cycle à 5 ou 6 chaînons, saturé ou non, hétérocyclique ou non, l'hétéroatome étant choisi parmi O, S et N, le système cyclique pouvant être substitué ou non par

des atomes d'halogène, des radicaux alkyles ou halogénoalkyles en $C_1$ à $C_4$, linéaires ou ramifiés.

3. Composés selon la revendication 1 ou 2 caractérisés en ce que Y représente

- le radical one-2 oxazolidinyl-3
- le radical one-2 oxazolidinyl-3 porteur de 1 à 4 substituants identiques ou différents choisis parmi :

. les radicaux alkyles en $C_1$ à $C_8$, linéaires ou ramifiés, substitués ou non par des atomes de fluor, chlore ou brome, le radical méthylthio, le groupe hydroxyle,

. les radicaux méthoxy et éthoxy,

. les radicaux alkoxycarbonyle en $C_1$ à $C_4$,

. le groupe carboxyle,

. les radicaux cycloalkyle en $C_3$ ou $C_6$,

. le radical phényle porteur ou non d'un ou de plusieurs substituants choisis parmi les atomes de chlore, brome ou fluor, le groupe nitro ou cyano,les radicaux méthyle et méthoxy,

- le cycle one-2 oxazolidinyl-3 condensé avec un cycle à 6 chaînons saturé ou non, hétérocyclique ou non, l'hétéroatome étant choisi parmi O, S et N, le système cyclique étant substitué ou non par des groupes choisis parmi les atomes de fluor, chlore ou brome, les radicaux alkyles en $C_1$ à $C_3$, linéaires ou ramifiés, les radicaux mono, di- ou tri-, chloro-, bromo- ou fluorométhyle.

4. Composés selon l'une quelconque des revendications précédentes caractérisés en ce que Y représente le radical one-2 oxazolidinyl-3 substitué ou non par un à quatre groupes identiques ou différents, choisis parmi les radicaux alkyle en $C_1$ à $C_4$, linéaires ou ramifiés.

5. Composés de formule Y-S-Cl (III), dans laquelle Y est telle que définie dans les revendications 1 à 4.

6. Procédé de préparation des composés de formule Y-S-Cl (III), tels que définis dans la revendication précédente caractérisé en ce que l'on fait réagir un composé de formule Y-H dans laquelle Y à la signification précédente avec le dichlorure de soufre, $SCl_2$, en présence d'un composé basique, éventuellement dans un milieu solvant inerte vis à vis des réactifs.

7. Procédé selon la revendication précédente caractérisé en ce que le composé basique est une amine tertiaire telle que la pyridine, la triéthylamine, la lutidine, la picoline, la N,N-diméthylaniline.

8. Procédé de préparation des composés de formule (III), Y-S-Cl tels que définis dans la revendication 5 caractérisé en ce que l'on fait réagir un dérivé N-trialkylsilylé d'un composé de formule Y-H, le radical alkyl ayant de 1 à 4 atomes de carbone et Y ayant la signification précédente, avec le dichlorure de soufre, $SCl_2$, éventuellement dans un milieu solvant inerte vis à vis des réactifs.

9. Procédé selon l'une quelconque des revendications 6 à 8 caractérisé en ce que les solvants sont choisis parmi les solvants aliphatiques halogénés, les éthers, les solvants aromatiques.

10. Procédé selon l'une quelconque des revendications 6 à 9 caractérisé en ce que la température de la réaction est comprise entre - 10° et 50° C et de préférence entre 0° et 20° C.

11. Procédé selon l'une quelconque des revendications 6 à 10 caractérisé en ce que $SCl_2$ est utilisé en léger excès.

12. Procédé de préparation des composés de formule (I) tels que définis dans les revendications 1 à 4 caractérisé en ce que l'on fait réagir le composé de formule

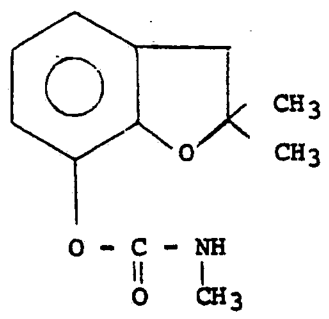

(II)

avec un composé de formule Y-S-Cl (III), dans laquelle Y est telle que définie dans les revendications 1 à 4 en présence d'un composé basique.

13. Procédé selon la revendication précédente caractérisé en ce qu'on effectue la réaction dans un milieu solvant inerte vis à vis des réactifs.

14. Procédé selon la revendication précédente caractérisé en que le solvant est choisi parmi les solvants aliphatiques chlorés, les solvants aromatiques, les éthers cycliques.

15. Procédé selon la revendication 13 ou 14 caractérisé en ce que le milieu solvant est anhydre.

16. Procédé selon l'une quelconque des revendications 12 à 15 caractérisé en ce que le composé basique est choisi parmi les amines tertiaires, les bases pyridiniques, les bases minérales telles que les hydroxydes et carbonates alcalins.

17. Procédé selon l'une quelconque des revendications 12 à 16 caractérisé en ce que la quantité du composé basique est comprise entre 1 et 10 moles par mole du composé de formule (II) et de préférence entre 1 et 1,6 moles.

18. Procédé selon l'une quelconque des revendications 12 à 17 caractérisé en ce que l'on utilise une quantité du composé (III) comprise entre 1 et 2 moles par mole du composé de formule (II), de préférence entre 1 et 1,2 moles.

19. Procédé selon l'une quelconque des revendications 12 à 18 caractérisé en ce que la température de la réaction est comprise entre - 10 °C et 50 °C, de préférence entre 0 ° et 35 °C.

20. Composition pesticide caractérisée en ce qu'elle contient comme matière active une quantité efficace comme pesticide d'un composé tel que défini dans l'une quelconque des revendications 1 à 4.

21. Composition pesticide caractérisée en ce qu'elle contient comme matière active de 0,1 % à 99% d'un composé tel que défini dans l'une quelconque des revendications 1 à 4.

22. Procédé de lutte contre les différents types d'insectes, les acariens, les myriapodes, les nématodes, les parasites des plantes et des animaux, caractérisé en ce qu'il consiste à leur appliquer une dose efficace d'un composé tel que défini dans les revendications 1 à 4.

23. Procédé de lutte contre les différents types d'insectes, les acariens, les myriapodes, les nématodes, les parasites des plantes, caractérisé en ce qu'il consiste à leur appliquer une dose efficace d'un composé tel que défini dans les revendications 1 à 4, soit par voie foliaire, soit par application au sol.

Office européen des brevets

# RAPPORT DE RECHERCHE EUROPEENNE

Numero de la demande

EP 89 40 3374

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF AGRICULTURAL AND FOOD CHEMISTRY, vol. 26, no. 3, 1978, pages 550-557, American Chemical Society, Washington, DC, US; M.A.H. FAHMY et al.: "Selective toxicity of N,N'-thiodicarbamates"<br>* En entier, page 552, tableau II, composés 1-12; page 554, tableau III, composés 1-12 *<br>----- | 1-23 | C 07 D 413/12<br>A 01 N 47/24<br>C 07 D 263/26 |

DOMAINES TECHNIQUES RECHERCHES (Int. Cl.5)

C 07 D 413/00
C 07 D 263/00

Le présent rapport a été établi pour toutes les revendications

| Lieu de la recherche | Date d'achèvement de la recherche | Examinateur |
|---|---|---|
| LA HAYE | 21-03-1990 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)